(19) Europäisches Patentamt / European Patent Office / Office européen des brevets

(11) **EP 1 855 740 B1**

(12) **EUROPÄISCHE PATENTSCHRIFT**

(45) Veröffentlichungstag und Bekanntmachung des Hinweises auf die Patenterteilung:
**11.03.2009 Patentblatt 2009/11**

(21) Anmeldenummer: **06700018.2**

(22) Anmeldetag: **11.01.2006**

(51) Int Cl.:
*A61M 5/315* (2006.01)    *A61M 5/24* (2006.01)

(86) Internationale Anmeldenummer:
**PCT/CH2006/000019**

(87) Internationale Veröffentlichungsnummer:
**WO 2006/089437 (31.08.2006 Gazette 2006/35)**

(54) **ZAHNEINGRIFF MIT UNGLEICHER TEILUNG**

TOOTH MESHING WITH UNEVEN DISTRIBUTION

ENGRENEMENT A REPARTITION INEGALE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **22.02.2005 DE 102005008065**

(43) Veröffentlichungstag der Anmeldung:
**21.11.2007 Patentblatt 2007/47**

(73) Patentinhaber: **TecPharma Licensing AG
3401 Burgdorf (CH)**

(72) Erfinder:
• **BURREN, Stefan
CH-3047 Bremgarten (CH)**
• **MOSER, Ulrich
CH-3412 Heimiswil (CH)**

(56) Entgegenhaltungen:
EP-A- 1 228 777          WO-A-00/23133
DE-A1-3102004 011 49    US-A- 2 221 739
US-A- 3 517 668          US-A1- 2001 051 792

EP 1 855 740 B1

**Beschreibung**

Die vorliegende Erfindung bezieht sich auf eine Dosiervorrichtung zur dosierten Verabreichung eines injizierbaren Produkts, insbesondere im medizinischen Bereich eine Injektionsvorrichtung, mit einer Zahnstange mit einer Vielzahl an Sperrgliedern sowie mit Sperrelementen, wobei mit der vorliegenden Dosiervorrichtung zum Beispiel feine Dosiermengen eines injizierbaren Produkts eingestellt werden können, die üblicherweise mit Zahnstangen mit kleineren Abständen zwischen den Sperrgliedern erfordern.

Die EP 1 228 777 B1 beschreibt eine Injektionseinheit mit welcher mehrere Injektionen verabreicht werden könne, bei welcher die Sperrglieder der Zahnstange auf gegenüberliegenden Seiten in Längsrichtung der Zahnstange versetzt zueinander angeordnet sind, wodurch eine verfeinerte Einstellung einer Dosiermenge eines injizierbaren Produkts erreicht wird.

Die US2221739A beschreibt eine Spritze, um eine kontrollierte Dosiermenge eines Betäubungsmittels zu verabreichen, wobei der Benutzer einen Hebel zu betätigen hat. Durch die Hebelbewegung greifen Zähne des Hebels abwechselnd in Lücken einer Zahnstange ein und geben die Zahnstange in die Vorschubrichtung frei.

Die US20010051792A1 offenbart eine Injektionsvorrichtung, mit welcher eine exaktere Einstellung einer Dosiermenge vollführt werden kann, bei welcher verschiedene Serien von Zähnen an einer Zahnstange angeordnet sind.

Die US3517668A beschreibt eine Injektionspistole mit schwenkbar an einem Hebel angeordneten Zähnen, die in eine Zahnstange eingreifen können, um eine Injektion auszulösen.

WO00/23133A offenbart ein Injektionsgerät mit federnden Teilen, die so ausgebildet sind, dass sie mit den an einer Zahnstange angebrachten Zähnen in Eingriff kommen.

Es ist eine Aufgabe der vorliegenden Erfindung, eine Vorrichtung und ein Verfahren zur Vorbereitung der dosierten Verabreichung eines injizierbaren Produkts aus einer Injektionsvorrichtung vorzuschlagen, mit welchen kleinere Dosiermengen eines injizierbaren Produkts eingestellt und verabreicht werden können.

Die erfindungsgemäße Dosiervorrichtung zur Verabreichung eines injizierbaren Produkts aus einer Injektionsvorrichtung umfasst eine Zahnstange, die eine Vielzahl an Sperrgliedern, insbesondere an sägezahnförmig ausgebildeten Zähnen oder Nocken, aufweist, wobei die Sperrglieder entlang der Längsachse der Zahnstange ausgebildet sind und jeweils einen Abstand voneinander aufweisen, der konstant oder von Sperrglied zu Sperrglied verschieden sein kann. Weiter umfasst die erfindungsgemäße Vorrichtung mindestens zwei Sperrelemente, die zum Beispiel an einem Führungsstück und/oder an einer Vorschubhülse oder an einem Schnapper oder Rastelement eines Führungsstücks oder einer Vorschubhülse der Injektionsvorrichtung angeordnet sein können, und entlang der Längsachse der Zahnstange der Injektionsvorrichtung einen Abstand voneinander aufweisen, wobei in dem Falle, wenn mehr als zwei Sperrelemente an der Injektionsvorrichtung angeordnet sind, die jeweiligen Abstände zwischen den Sperrelementen in Längsrichtung der Zahnstange konstant sein können oder unterschiedlich sein können. Erfindungsgemäß sind der erste konstante Abstand zwischen den Sperrgliedern der Zahnstange oder die ersten Abstände zwischen den Sperrgliedern der Zahnstange, wobei die jeweiligen Abstände zwischen den Sperrgliedern verschieden sein können, ungleich dem zweiten konstanten Abstand zwischen den Sperrelementen oder den zweiten Abständen zwischen den Sperrelementen, wobei die jeweiligen Abstände zwischen den Sperrelementen verschieden sein können.

Insbesondere können der erste Abstand oder die jeweiligen ersten Abstände zwischen den Sperrgliedern der Zahnstange kleiner oder größer als der zweite Abstand oder die jeweiligen zweiten Abstände zwischen den Sperrelementen sein, wobei sich die Abstände vorzugsweise auf Abstände in Längsrichtung der Zahnstange oder der Injektionsvorrichtung beziehen.

Bevorzugt können die Sperrelemente oder Rastelemente, an denen die Sperrelemente angeordnet sein können, in Umfangsrichtung der Zahnstange an gleicher Position ausgebildet sein und zum Beispiel in Längsrichtung der Zahnstange durch einen zweiten konstanten Abstand oder unkonstante zweite Abstände versetzt zueinander oder getrennt voneinander angeordnet sein, so dass zum Beispiel nur auf einer Seite der Zahnstange Sperrelemente vorgesehen sein können, welche in die Sperrglieder der Zahnstange eingreifen können. Auch können die Sperrelemente in Umfangsrichtung der Zahnstange versetzt oder voneinander beabstandet angeordnet sein, wobei sich insbesondere Sperrelemente gegenüberliegen können, und die Sperrelemente können in Längsrichtung der Zahnstange an gleicher Position und/oder versetzt zueinander angeordnet sein, so dass sich zum Beispiel Sperrelemente gegenüberliegen können und in Längsrichtung an gleicher Position angeordnet sein können oder in Umfangsrichtung und Längsrichtung versetzt zueinander angeordnet sein können.

Insbesondere können zwei, drei, vier, fünf, sechs, sieben, acht oder mehr als acht Sperrelemente an der Injektionsvorrichtung vorgesehen sein, wobei vorzugsweise eine geradzahlige Anzahl an Sperrelementen an der Injektionsvorrichtung angeordnet ist, wobei bevorzugt in Umfangsrichtung an gleicher Position und in Längsrichtung hintereinander und diesen Sperrelementen zum Beispiel gegenüberliegend an gleicher Position in Längsrichtung wiederum Sperrelemente gleicher Anzahl angeordnet sein können.

Insbesondere können die Sperrelemente in Längsrichtung der Zahnstange einen konstanten Abstand voneinander aufweisen, wobei nicht jedes der Sperrelemente in die Sperrglieder der Zahnstange einrastet, sondern zum Beispiel jedes zweite Sperrelement in Längsrichtung der Zahnstange in die Sperrglieder der Zahnstange einrastet. Dabei ist unter. Einrasten im Sinne der Erfindung zu verstehen, dass die Sperrelemente mit ihrer Anschlagsflänke, also der Flanke der Sperrelemente oder Nocken, in dem Zustand, in dem die Sperrelemente zwischen den Sperrgliedern der Zahnstange liegen, senkrecht auf der Zahnstange steht, an einem Gegenanschlag eines Sperrglieds anliegen, wobei der Gegenanschlag die Flanke der Sperrglieder oder Zähne der Zahnstange darstellt, welche senkrecht auf der Zahnstange steht. Die weiteren Sperrelemente rasten dabei nicht in die Sperrglieder der Zahnstange ein, liegen also nicht mit ihrer Anschlagsflanke an einem Gegenanschlag eines Sperrglieds an. Aufgrund des unterschiedlichen Abstands zwischen den Sperrgliedern der Zahnstange verglichen mit dem Abstand zwischen den Sperrelementen, der von einem Sperrelement zum nächsten sowohl konstant als auch unkonstant sein kann, liegt nicht jede Anschlagsflanke eines Sperrelements an einem Gegenanschlag eines Sperrglieds an, sondern zum Beispiel nur jede zweite, dritte, vierte, fünfte oder sechste Anschlagsflanke eines Sperrelements, wobei die übrigen oder weiteren Sperrelemente nicht mit den Sperrgliedern der Zahnstange verrasten, also nicht mit ihrer Anschlagsflanken an einem Gegenanschlag eines Sperrglieds anliegen.

Soll zum Beispiel jedes n-te Sperrelement in die Zahnung der Zahnstange einrasten, also jedes n-te Sperrelement mit seiner Anschlagsflanke an einem Gegenanschlag eines Sperrglieds der Zahnstange anliegen, die weiteren oder übrigen Sperrelemente jedoch nicht in die Zahnstange einrasten, also mit ihrer Anschlagsflanke nicht an einem Gegenanschlag eines Sperrglieds der Zahnstange anliegen, so können die hierfür notwendigen jeweiligen Abstände zwischen den Sperrelementen in Abhängigkeit des Abstands zwischen den Sperrgliedern der Zahnstange berechnet werden.

Ist der erste Abstand zwischen den Sperrgliedern der Zahnstange kleiner als der zweite Abstand zwischen den Sperrelemente, so kann der jeweilige Abstand zwischen den Sperrelementen in Abhängigkeit des Abstands zwischen den Sperrgliedern der Zahnstange dargestellt werden als:

$$A2 = A1 \cdot \frac{n \cdot k + 1}{n}; k \in N \wedge n \in \mathrm{N}$$

Dabei kann der Wert k, der aus der Menge der natürlichen Zahlen gewählt werden kann, bei jeder Berechnung eines Abstands der Sperrelemente voneinander verändert werden, wobei sich bei gleich bleibendem Wert von k konstante Abstände zwischen den aufeinander folgenden Sperrelementen ergeben. Durch den Wert n, der aus der Menge der natürlichen Zahlen gewählt werden kann, wird festgelegt, jedes wievielte Sperrelement mit seiner Anschlagsflanke an einem Gegenanschlag der Zahnstange anliegt, um eine Kraftübertragung auf die Zahnstange durchzuführen oder um die Zahnstange zu bewegen oder eine Bewegung der Zahnstange zu verhindern. Erweitert man den Wertebereich von k auf $k \in N_O$, so kann auch der Spezialfall berücksichtigt werden, dass bei k=O die Abstände zwischen den Sperrelementen minimal werden und nur von dem Wert von n abhängen.

Ist der erste Abstand zwischen den Sperrgliedern der Zahnstange größer als der zweite Abstand zwischen den Sperrelementen ergibt sich der Abstand zwischen den Sperrelementen in Abhängigkeit des Abstands zwischen den Sperrgliedern der Zahnstange als:

$$A2 = A1 \cdot \frac{n \cdot k - 1}{n}; k \in N \wedge n \in N$$

Die Sperrelemente der erfindungsgemäßen Injektionsvorrichtung sind vorzugsweise unelastisch und als sägezahnförmige Nocken oder Zähne ausgebildet. Die Nocken oder Zähne weisen insbesondere eine Anschlagsflanke und eine Gleitflanke auf, wobei als Anschlagsflanke die Flanke der Nocken bezeichnet wird, welche senkrecht auf der Zahnstange steht, wenn sich die Sperrelemente zwischen den Sperrgliedern der Zahnstange befinden. In dieser Position bezeichnet die Gleitflanke die schräg auf der Zahnstange stehende Flanke des Nockens oder des Sperrelements.

Insbesondere können die Sperrelemente in radialer Richtung relativ zu der Zahnstange bewegt werden oder sie können an einem Rastelement oder einem Schnapper ausgebildet sein, welcher relativ zu der Zahnstange in radialer Richtung bewegt werden kann. Auch können die Sperrelemente an einer Vorschubhülse vorgesehen sein, welche relativ zu der Zahnstange bewegt werden kann oder die Sperrelemente können an einem Führungsstück vorgesehen sein, wobei die Zahnstange relativ zu dem Führungsstück bewegt werden kann. Auch können die Vorschubhülse und das Führungsstück, an welchem die Sperrelemente angeordnet sein können, zum Beispiel in radialer Richtung relativ zu der Zahnstange bewegt werden.

Die Sperrglieder der Zahnstange können zum Beispiel über die gesamte Länge der Zahnstange ausgebildet sein und zum Beispiel entlang der Längsachse der Zahnstange konstante Abstände voneinander aufweisen oder sie können abschnittweise konstante Abstände voneinander aufweisen, indem die Sperrglieder der Zahnstange in einem ersten Abschnitt entlang der Längsachse der Zahnstange jeweils einen ersten konstanten Abstand voneinander aufweisen und in einem auf den ersten Abschnitt folgenden oder angrenzenden oder benachbarten zweiten Abschnitt entlang der Längsachse der Zahnstange jeweils einen zweiten konstanten Abstand voneinander aufweisen, wobei der erste Abstand der Sperrglieder in dem ersten Abschnitt und der zweite Abstand der Sperrglieder in dem zweiten Abschnitt unterschiedlich sein können. Auch können mehr als zwei Abschnitte mit unterschiedlichen Abständen zwischen den Sperrgliedern aufeinander folgen oder es können sich zum Beispiel Abschnitte mit jeweils unterschiedlichen Abständen zwischen den Sperrglieder abwechseln oder periodisch abwechseln.

Bei dem erfindungsgemäßen Verfahren zur Vorbereitung der dosierten Verabreichung eines injizierbaren Produkts aus einer Injektionsvorrichtung wird zunächst eine erste Aufziehbewegung der Injektionsvorrichtung durch die Bewegung eines Einstellmittels oder Knopfes entgegen der Ausschüttrichtung des injizierbaren Produkts durchgeführt, wobei durch die Bewegung des Einstellmittels oder Knopfes eine Vorschubhülse entgegen der Ausschüttrichtung des injizierbaren Produkts relativ zu einer Zahnstange bewegt wird. Da mindestens ein Sperrelement eines Führungsstücks mit Sperrgliedern der Zahnstange verrastet ist, indem zum Beispiel eine Anschlagsflanke des mindestens einen Sperrelements des Führungsstücks an einem Gegenanschlag eines Sperrglieds der Zahnstange anliegt, wird die Zahnstange durch das Führungsstück in Ruhe gehalten. Dabei liegt die Anschlagsflanke mindestens eines Sperrelements des Führungsstücks nicht an einem Gegenanschlag eines Sperrglieds der Zahnstange an, ist also nicht zum Beispiel mit der Zahnung der Zahnstange verrastet. Nach Beendigung der Aufziehbewegung liegt eine Anschlagsflanke mindestens eines Sperrelements der Vorschubhülse an einem Gegenanschlag eines Sperrglieds der Zahnstange an, wobei durch die Bewegung des Einstellelementes oder Knopfes in Ausschüttrichtung des injizierbaren Produkts die Vorschubhülse in Ausschüttrichtung bewegt wird und über die anliegende Anschlagsflanke oder die anliegenden Anschlagflanken auf einen Gegenanschlag eines Sperrglieds der Zahnstange eine Kraft übertragen wird, wodurch die Zahnstange in Ausschüttrichtung relativ zu dem Führungsstück bewegt wird. Dabei liegt eine Anschlagsflanke mindestens eines. Sperrelements der Vorschubhülse nicht an einem Gegenanschlag eines Sperrglieds der Zahnstange an. Eine erste Dosis des injizierbaren Produkts wird durch die Verschiebung der Zahnstange abgegeben, wobei nach der Relativbewegung zwischen Zahnstange und Führungsstück eine Anschlagsflanke mindestens eines Sperrelements des Führungsstücks an einem Gegenanschlag eines Sperrglieds der Zahnstange anliegt und eine Anschlagsflanke mindestens eines Sperrelements des Führungsstücks nicht an einem Gegenanschlag eines Sperrglieds der Zahnstange anliegt. Darauf kann ein erneuter Aufziehvorgang und darauf folgender Abgabevorgang des injizierbaren Produkts durchgeführt werden.

Insbesondere kann bei dem erfindungsgemäßen Verfahren jedes n-te Sperrelement mit seiner Anschlagsflanke an einem Gegenanschlag eines Sperrglieds der Zahnstange anliegen und zum Beispiel eine Kraft auf die Zahnstange übertragen, um zum Beispiel die Zahnstange zu bewegen, oder eine Kraft von der Zahnstange aufnehmen, um beispielsweise eine Bewegung der Zahnstange zu verhindern, wobei die weiteren oder übrigen Sperrelemente nicht mit der Zahnstange verrastet sind, also zum Beispiel nicht mit ihrer Anschlagsflanke an einem Gegenanschlag eines Sperrglieds der Zahnstange anliegen. Nach dem Aufziehen der Injektionsvorrichtung entgegen der Ausschüttrichtung des injizierbaren Produkts und der mit dem Aufziehen verbundenen Bewegung der Vorschubhülse relativ zu der Zahnstange kann jedes (n+1)-te Sperrelement der Vorschubhülse mit seiner Anschlagsflanke an einem Gegenanschlag eines Sperrglieds der Zahnstange anliegen, wobei die weiteren Sperrelemente der Vorschubhülse mit ihrer Anschlagsflanke nicht an einem Gegenanschlag eines Sperrglieds der Zahnstange anliegen.

Vor der Ausschüttung des injizierbaren Produkts kann zum Beispiel jedes n-te Sperrelement des Führungsstücks mit seiner Anschlagsflanke an einem Gegenanschlag eines Sperrglieds der Zahnstange anliegen, wobei die weiteren Sperrelemente des Führungsstücks nicht mit ihrer Anschlagsflanke an einem Gegenanschlag eines Sperrglieds der Zahnstange anliegen, wobei nach der Durchführung der Ausschüttung des injizierbaren Produkts aufgrund der Bewegung der Zahnstange relativ zu dem Führungsstück vorzugsweise jedes (n+1)-te Sperrelement des Führungsstücks mit den Sperrgliedern der Zahnstange verrastet ist und die weiteren oder übrigen Sperrelemente des Führungsstücks nicht mit den Sperrgliedern der Zahnstange verrastet sind.

Insbesondere kann auch nach jedem Aufziehvorgang durch Bewegung des Einstellelementes oder Knopfes in Ausschüttrichtung des injizierbaren Produkts eine konstante Abgabemenge des injizierbaren Produkts ausgeschüttet werden.

Die Erfindung wird nachfolgend anhand bevorzugter Ausführungsbeispiele beschrieben werden. Es zeigen:

Figur 1      eine erste Ausführungsform der vorliegenden Erfindung mit jeweils zwei Sperrelementen an sich gegenüberliegenden Armen des Führungsstücks und der Vorschubhülse mit kleinerem Abstand zwischen den Sperrgliedern der Zahnstange als dem Abstand zwischen den Sperrelementen;

Figur 2      die Injektionsvorrichtung aus Figur 1 mit jeweils 4 Eingriffselementen an den beiden sich gegenüberliegenden Armen des Führungsstücks und der Vorschubhülse;

Figur 3a      Gesamtdarstellung der Injektionsvorrichtung aus Figur 2;

Figur 3b      die Injektionsvorrichtung aus Figur 3a vor dem Aufziehen der Injektionsvorrichtung und vor dem Abgeben eines injizierbaren Produkts;

Figur 3c      die Injektionsvorrichtung aus den Figuren 3a und 3b nach dem Abgeben eines injizierten Produkts;

Figur 4      eine zweite Ausführungsform der vorliegenden Erfindung mit jeweils drei Eingriffselementen an den beiden sich gegenüberliegenden Armen des Führungsstücks und der Vorschubhülse mit größerem Abstand zwischen den Sperrgliedern der Zahnstange verglichen mit dem Abstand zwischen den Sperrelementen;

Figuren 5a-5c      eine schematische Darstellung der Zahnstange und der Sperrelemente mit verschiedenen Abständen zwischen den Sperrelementen für den Fall n=2.

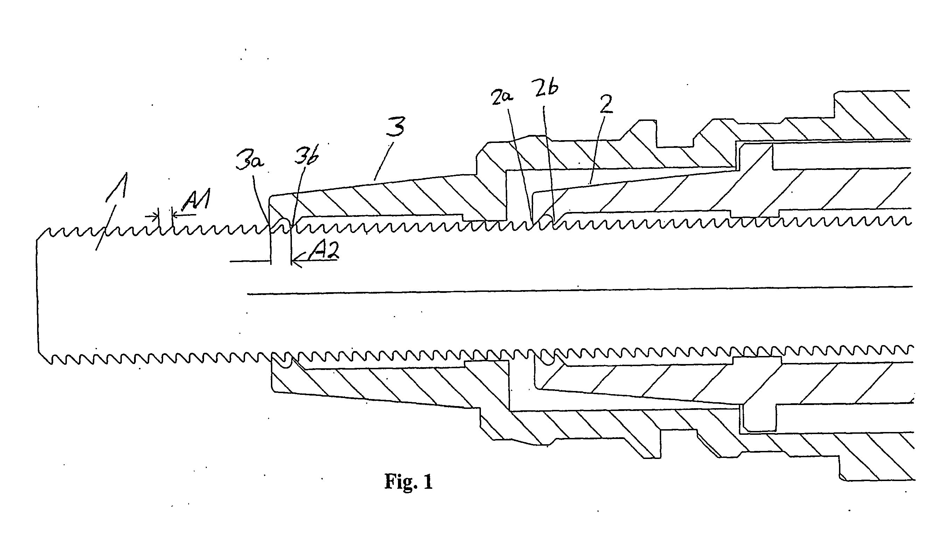

Figur 1 zeigt eine erste Ausführungsform der vorliegenden Erfindung mit einer Zahnstange 1, einer relativ zur Zahnstange 1 verschiebbaren Vorschubhülse 2 und einem Führungsstück 3, wobei die Zahnstange 1 relativ zu dem Führungsstück 3 bewegt werden kann. An den zwei sich gegenüberliegenden Armen der Vorschubhülse 2 und an den sich gegenüberliegenden Armen des Führungsstücks 3 sind jeweils zwei Sperrelemente 2a, 2b, 3a, 3b angeordnet, wobei der Abstand A2 zwischen den Sperrelementen 2a, 2b, 3a, 3c größer ist als der Abstand A1 zwischen den Sperrgliedern der Zahnstange 1. Dabei liegt die Anschlagsflanke des zweiten oder in Ausschüttrichtung hinteren Sperrelements 2b der Vorschubhülse 2 an einem Gegenanschlag eines Sperrglieds der Zahnstange 1 an, so dass zum Beispiel durch eine Vorschubbewegung der Vorschubhülse 2 eine Kraft in Ausschüttrichtung von dem hinteren Sperrelement 2b, welches mit seiner Anschlagsflanke an einem Gegenanschlag eines Sperrglieds der Zahnstange anliegt, auf die Zahnstange 1 übertragen werden kann, um zum Beispiel die Zahnstange 1 in Ausschüttrichtung zu bewegen. In der vorliegenden Ausführungsform kann die Vorschubhülse 2 und damit die Zahnstange 1 so weit in Ausschüttrichtung bewegt werden, bis die Vorschubhülse 2 mit ihrem Anschlag an dem Führungsstück 3 anliegt. Eine Dosis des injizierbaren Produkts kann eingestellt werden, indem zum Beispiel die Vorschubhülse 2 entgegen der Ausschüttrichtung bewegt wird, wobei die Zahnstange in Ruhe bleibt, da sie durch das Führungsstück 3 gehalten wird. Insbesondere wird die Zahnstange 3 durch das vordere Sperrelement 3a des Führungsstücks 3 gehalten, das mit seiner Anschlagsflanke an einem Gegenanschlag eines Sperrglieds der Zahnstange 1 anliegt, wobei das hintere Sperrelement 3b des Führungsstücks 3 nicht mit seiner Anschlagsflanke an einem Gegenanschlag eines Sperrglieds der Zahnstange 1 anliegt. Mit der vorliegenden Ausführungsform der Erfindung können sehr feine Dosierungen erreicht werden, indem zum Beispiel die Vorschubhülse 2 entgegen der Ausschüttrichtung bewegt wird und eine Aufziehbewegung ausgeführt wird, wobei nach der Bewegung der Vorschubhülse 2 um ein Sperrglied oder einen Klick weiter entgegen der Ausschüttrichtung nicht mehr das hintere 2b sondern das vordere Sperrelement 2a der Vorschubhülse 2 mit seiner Anschlagsflanke an einem Gegenanschlag eines Sperrglieds der Zahnstange 1 anliegen würde, und eine Kraft von der Vorschubhülse 2 auf die Zahnstange 1 in Ausschüttrichtung übertragen könnte, um einen Ausschüttvorgang durchzuführen.

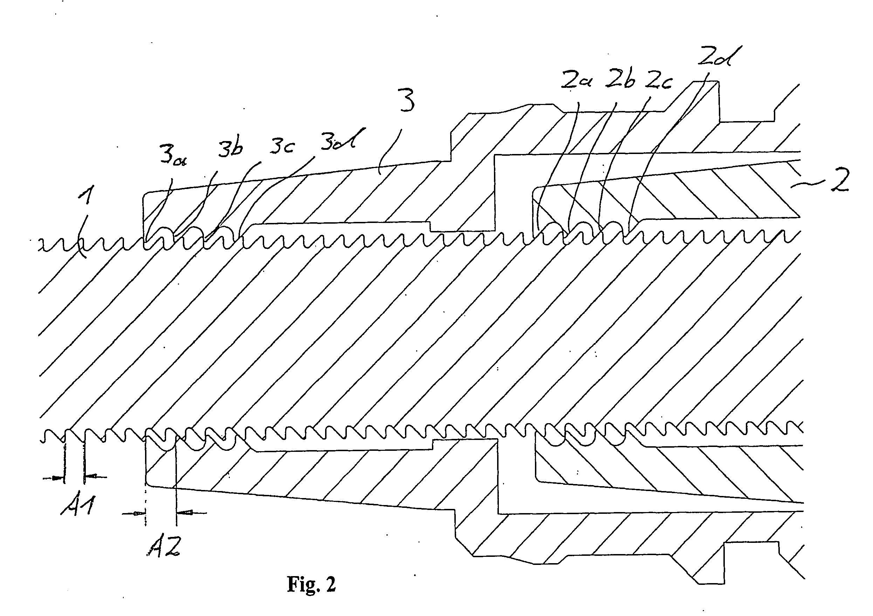

Figur 2 zeigt die Injektionsvorrichtung aus Figur 1 in einer weiteren Ausführungsform, wobei nunmehr an jedem Arm der Vorschubhülse 2 und des Führungsstücks 3 vier Sperrelemente 2a, 2b, 2c, 2d, 3a, 3b, 3c, 3d angeordnet sind, wobei jedes zweite Sperrelement des Führungsstücks 3 und der Vorschubhülse 2 in die Sperrglieder oder die Zähne der Zahnstange 1 eingreift und jedes zweite Sperrelement des Führungsstücks 3 und der Vorschubhülse 2 nicht in die Zahnstange 1 eingreift. Dabei liegt das zweite und vierte Sperrelement 2b, 2d der Vorschubhülse 2 mit seiner Anschlagsflanke an einem Gegenanschlag eines Sperrglieds an, um eine Kraft auf die Zahnstange 1 übertragen zu können oder die Zahnstange 1 zu bewegen, und jedes erste 3a und dritte 3c Sperrelement des Führungsstücks 3 liegt mit seiner Anschlagsflanke an einem Gegenanschlag eines Sperrglieds der Zahnstange 1 an, um eine Kraft von der Zahnstange 1 aufnehmen zu können oder eine Bewegung der Zahnstange 1 zu verhindern. Mit der Injektionsvorrichtung aus Figur 2 können die gleichen Dosiermengen eingestellt werden wie mit der Injektionsvorrichtung aus Figur 1, wobei auf Grund der vier Sperrelemente an den Armen der Vorschubhülse 2 und des Führungsstücks 3 eine hohe Stabilität der Zahnstange 1 und damit eine genaue Bewegung der Zahnstange 1 erreicht werden kann.

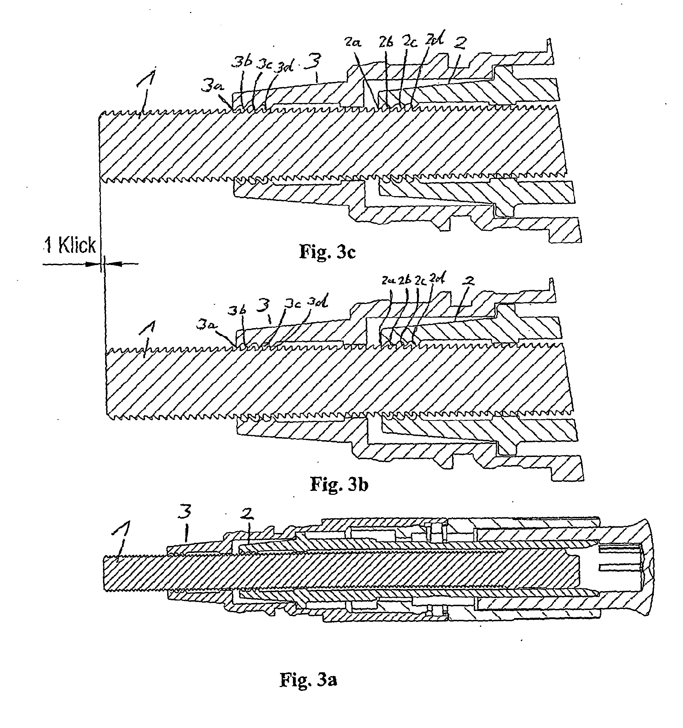

Figur 3a zeigt die Injektionsvorrichtung aus Figur 2 in einer Gesamtansicht während die Figuren 3b und 3c diese Injektionsvorrichtung in zwei verschiedenen Zuständen zeigen. Figur 3a zeigt die Injektionsvorrichtung in einer Grundstellung vor einem Aufzieh- und Ausschüttvorgang, wobei jeweils das erste 2a und dritte Sperrelement 2c der Vorschubhülse 2 an einem Sperrglied der Zahnstange 1 anliegt oder mit der Zahnstange 1 verrastet ist. So kann zum Beispiel eine Aufziehbewegung der Injektionsvorrichtung durchgeführt werden, indem zum Beispiel ein Knopf oder Einstellelement entgegen der Ausschüttrichtung bewegt wird und die Vorschubhülse 2 entgegen der Ausschüttrichtung um ein Sperrglied oder einen Klick weiter bewegt wird, so dass nun das zweite 2b und vierte Sperrelement 2d der Vorschubhülse 2 an einem Sperrglied der Zahnstange 1 anliegt, wie in Figur 3b dargestellt, da die Zahnstange 1 in Ruhe bleibt und somit eine Relativbewegung der Vorschubhülse 2 relativ zu der Zahnstange 1 durchgeführt wird. Die Zahnstange 1 wird während der Bewegung der Vorschubhülse 2 entgegen der Ausschüttrichtung durch das Führungsstück 3, insbesondere durch das zweite 3b und vierte Sperrelement 3d des Führungsstücks 3, wie in Figur 3a gezeigt, in Ruhe gehalten, da das zweite 3b und vierte Sperrelement 3d mit ihrer Anschlagsflanke an einem Gegenanschlag von Sperrgliedern anliegen, und somit eine Bewegung der Zahnstange 1 verhindern. Nach dem Aufziehen der Injektionsvorrichtung kann eine Ausschüttung des injizierbaren Produkts durchgeführt werden, indem zum Beispiel ein Knopf oder Einstellelement und damit die Vorschubhülse 2 in Ausschüttrichtung bewegt wird und zum Beispiel das zweite 2b und das vierte Sperrelement 2d der Vorschubhülse 2 eine Kraft in Ausschüttrichtung auf die Zahnstange 1 übertragen, wodurch eine Bewegung der Zahnstange 1 in Ausschüttrichtung und damit eine Ausschüttung des injizierbaren Produkts durchgeführt wird, wobei sich die Zahnstange 1 relativ zu dem Führungsstück 3 bewegt. Wird die Zahnstange 1 um ein Sperrglied oder einen Klick in Ausschüttrichtung relativ zu dem Führungsstück 3 bewegt, so liegen das erste 3a und dritte Sperrelement 3c des Führungsstücks 3 an Sperrgliedern der Zahnstange 1 an, wobei durch die Bewegung um einen Klick eine geringe Dosis abgegeben wird, insbesondere ein kürzerer Weg der Zahnstange 1 in Ausschüttrichtung zurückgelegt wird, als der Weg oder Abstand zwischen zwei Sperrgliedern oder zwischen zwei Sperrelementen beträgt.

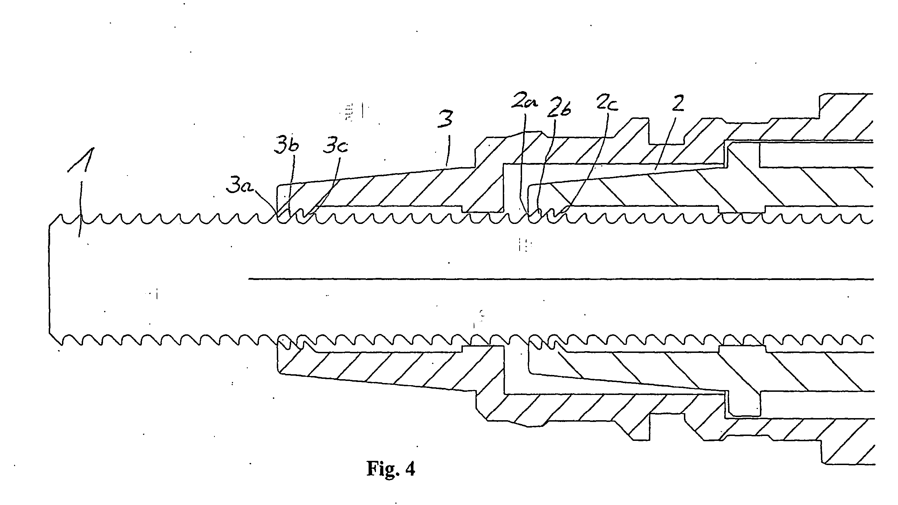

Figur 4 zeigt eine weitere Ausführungsform der vorliegenden Erfindung, wobei im Gegensatz zu der Injektionsvorrichtung aus Figur 1 die Abstände zwischen den Sperrgliedern der Zahnstange 1 größer sind als die Abstände zwischen den Sperrelementen 2a, 2b, 2c, 3a, 3b, 3c. Dabei liegt jeweils nur das erste Sperrelement 2a, 3a des Führungsstücks 3 und der Vorschubhülse 2 an einem Gegenanschlag eines Sperrglieds oder Zahns der Zahnstange 1 an, während die übrigen Sperrelemente 2b, 2c, 3b, 3c nicht mit der Zahnstange 1 verrastet sind und somit keine Wirkverbindung zu der Zahnstange 1 besitzen, um zum Beispiel eine Kraft auf die Zahnstange 1 übertragen zu können. Wird zum Beispiel die Vorschubhülse 2 in Aufziehrichtung relativ zu der Zahnstange 1 bewegt, wobei die Zahnstange 1 durch das erste Sperrelement 3a des Führungsstücks 3 in Ruhe gehalten wird, wird nicht mehr das erste Sperrelement 2a der Vorschubhülse 2 eine Wirkverbindung zu der Zahnstange 1 besitzen, sondern wird das zweite Sperrelement 2b der Vorschubhülse 2 mit seiner Anschlagsflanke an einen Gegenanschlag eines Sperrglieds der Zahnstange 1 anliegen und eine Wirkverbindung zu der Zahnstange 1 ausbilden. Wird nun zum Beispiel die Vorschubhülse 2 in Ausschüttrichtung bewegt, überträgt der zweite Zahn 2b der Vorschubhülse 2 eine Kraft auf die Zahnstange 1 und bewegt sie relativ zu dem Führungsstück 3 um einen Klick oder ein Sperrglied weiter, so dass nach der Bewegung das zweite Sperrelement 3b des Führungsstücks 3 an einem Sperrglied der Zahnstange 1 anliegt. Durch die Bewegung der Zahnstange 1 relativ zu dem Führungsstück 3 kann zum Beispiel die Abgabe eines injizierbaren Produkts, wie einer Flüssigkeit, in kleinen Dosen erfolgen.

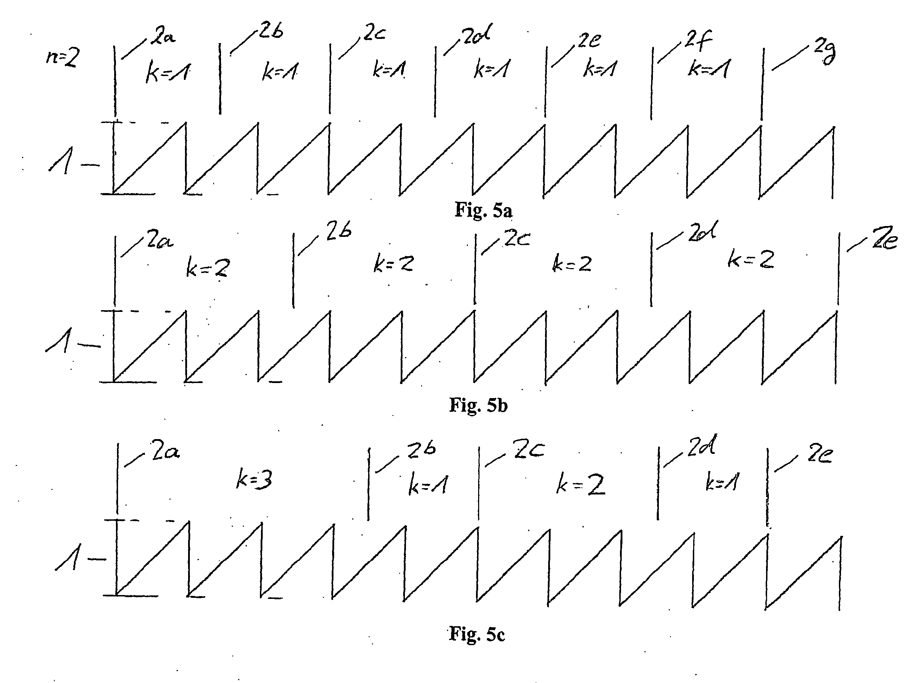

Figur 5a, 5b und 5c zeigen eine schematische Darstellung der Zahnstange 1 und der Sperrelemente für den Fall n=2, also für den Fall, bei dem jedes zweite Sperrelement mit seiner Anschlagsflanke an einem Gegenanschlag eines Sperrglieds der Zahnstange 1 anliegt, wobei in diesem Falle der Abstand der Sperrelemente größer ist als der Abstand zwischen den Sperrgliedern.

In Figur 5a wird bei der Berechnung jedes Abstandes der freie Wert k als 1 gewählt, so dass sich bei den Abständen zwischen den Sperrelementen der 1½-fache Abstand der Sperrglieder ergibt, also der Abstand zwischen den Sperrelementen das 3/2-fache des Abstands zwischen den Sperrgliedern beträgt. Dieselbe Funktionalität wie in Figur 5a und dieselbe Charakteristik, dass jedes zweite Sperrelement an einem Sperrglied anliegt, wird auch erreicht wenn der freie Parameter k als 2 gewählt wird, wodurch sich, wie in Figur 5b zu sehen, für den Abstand zwischen den Sperrelementen der 5/2-fache Abstand im Vergleich mit dem Abstand zwischen den Sperrgliedern der Zahnstange 1 ergibt. Auch hier kann eine Dosiermenge erreicht werden, welche einem halben Abstand zwischen den Sperrgliedern entspricht. Figur 5c verdeutlicht, dass der Abstand zwischen den Sperrelementen nicht konstant sein muss, sondern lediglich der

$$\text{Formel } A2 = A1 \cdot \frac{n \bullet k + 1}{n}$$ folgen muss, um dieselbe Funktionalität zu erreichen, wie zum Beispiel, dass Dosiermengen, die einem halben Abstand zwischen den Sperrgliedern entsprechen, eingestellt werden können. So entspricht in Figur 5c der Abstand zwischen dem ersten und dem zweiten Sperrelement bei einem Wert von k = 3 dem 7/2-fachen des Abstands zwischen den Sperrgliedern der Zahnstange 1, während der Abstand zwischen dem zweiten und dem dritten Sperrelement dem 3/2-fachen, der Abstand zwischen dem dritten und vierten Sperrelement dem 5/2-fachen und der Abstand zwischen dem vierten und fünften Sperrelement wieder dem 3/2-fachen des Abstands zwischen den Sperrgliedern entspricht, wobei auch andere ganzzahlige Werte für k beliebig verwendet werden können, um dieselbe Funktionalität oder Dosierung zu erreichen.

**Patentansprüche**

1. Dosiervorrichtung zur dosierten Verabreichung eines injizierbaren Produkts aus einer Injektionsvorrichtung mit:

- einer Zahnstange (1) mit einer Vielzahl an Sperrgliedern, wobei die Sperrglieder entlang der Längsachse der Zahnstange (1) einen ersten Abstand (A1) voneinander aufweisen; und
- mindestens zwei Sperrelementen (2a, 2b; 3a, 3b), wobei die Sperrelemente (2a, 2b; 3a, 3b) in Längsrichtung der Zahnstange (1) in einem festen Lageverhältnis relativ zueinander und in Umfangsrichtung der Zahnstange (1) an gleicher Position angeordnet sind und die Sperrelemente (2a, 2b; 3a, 3b) entlang der Längsachse der Zahnstange (1) einen zweiten Abstand (A2) voneinander aufweisen;

**dadurch gekennzeichnet, dass** der erste Abstand (A1) zwischen den Sperrgliedern der Zahnstange (1) ungleich dem zweiten Abstand (A2) zwischen den Sperrelementen (2a, 2b; 3a, 3b) ist.

2. Dosiervorrichtung nach dem vorhergehenden Anspruch, wobei der erste Abstand (A1) zwischen den Sperrgliedern der Zahnstange(1) kleiner ist als der zweite Abstand (A2) zwischen den Sperrelementen. (2a, 2b; 3a, 3b).

3. Dosiervorrichtung nach Anspruch 1, wobei der erste Abstand (A1) zwischen den Sperrgliedern der Zahnstange (1) grösser ist als der zweite Abstand (A2) zwischen den Sperrelementen (2a, 2b; 3a, 3b).

4. Dosiervorrichtung nach einem der vorhergehenden Ansprüche, wobei die Sperrelemente (2a, 2b; 3a, 3b) in Längsrichtung der Zahnstange (1) einen konstanten Abstand voneinander aufweisen.

5. Dosiervorrichtung nach einem der vorhergehenden Ansprüche, wobei zwei, drei, vier, fünf sechs, sieben, acht oder mehr Sperrelemente (2a, 2b; 3a, 3b) vorgesehen sind.

6. Dosiervorrichtung nach einem der vorhergehenden Ansprüche, wobei die Sperrelemente (2a, 2b; 3a, 3b) in Längsrichtung der Zahnstange (1) jeweils einen konstanten Abstand voneinander aufweisen, in Längsrichtung der Zahnstange (1) die Anschlagsflanke jedes zweiten, dritten, vierten, fünften oder sechsten Sperrelements (2a, 2b; 3a, 3b) an einem Gegenanschlag eines Sperrgliedes anliegt und die weiteren Sperrelemente (2a, 2b; 3a, 3b) mit ihrer Anschlagsflanke nicht an einem Gegenanschlag eines Sperrglieds der Zahnstange (1) anliegen.

7. Dosiervorrichtung nach einem der vorhergehenden Ansprüche, wobei jedes n-te Sperrelement (2a, 2b; 3a, 3b) mit seiner Anschlagsflanke an einem Gegenanschlag eines Sperrglieds der Zahnstange (1) anliegt, die weiteren Sperrelemente (2a, 2b; 3a, 3b) mit ihrer Anschlagsflanke nicht an einem Gegenanschlag eines Sperrglieds der Zahnstange (1) anliegen, der erste Abstand (A1) zwischen den Sperrgliedern der Zahnstange (1) kleiner ist als der zweite Abstand (A2) zwischen den Sperrelementen (2a, 2b; 3a, 3b) und der jeweilige Abstand (A2) zwischen den Sperrelementen (2a, 2b; 3a, 3b) in Abhängigkeit des ersten Abstands (A1) zwischen den Sperrgliedern der Zahnstange (1) dargestellt werden kann als:

$$A2 = \frac{n \cdot k + 1}{n} A1$$

wobei k und n aus der Menge der natürlichen Zahlen gewählt werden kann.

8. Dosiervorrichtung nach einem der vorhergehenden Ansprüche, wobei jedes n-te Sperrelement (2a, 2b; 3a, 3b) mit seiner Anschlagsflanke an einem Gegenanschlag eines Sperrglieds der Zahnstange (1) anliegt, die weiteren Sperrelementen (2a, 2b; 3a, 3b) mit ihrer Anschlagsflanke nicht an einem Gegenanschlag eines Sperrglieds der Zahnstange (1) anliegen, der erste Abstand (A1) zwischen den Sperrgliedern der Zahnstange (1) grösser ist als der zweite Abstand (A2) zwischen den Sperrelementen (2a, 2b; 3a, 3b) und der jeweilige Abstand (A2) zwischen den Sperrelementen (2a, 2b; 3a, 3b) und der jeweilige Abstand (A2) zsichen den Sperrelementen (2a, 2b; 3a, 3b) in Abhängigkeit des ersten Abstands (A1) zwischen den Sperrgliedern der Zahnstange (1) dargestellt werden kann als:

$$A2 = \frac{n \cdot k - 1}{n} A1$$

wobei k und n aus der Menge der natürlichen Zahlen gewählt werden kann.

9. Dosiervorrichtung nach einem der vorhergehenden Ansprüche, wobei die Sperrelemente (2a, 2b; 3a, 3b) als unelastische sägezahnförmige Nocken mit einer senkrechten Anschlagsflanke und einer schrägen Gleitflanke ausgebildet sind.

10. Dosiervorrichtung nach einem der vorhergehenden Ansprüche, wobei die Sperrelemente (2a, 2b; 3a, 3b) relativ zu der Zahnstange (1) in radialer Richtung bewegt werden können.

11. Dosiervorrichtung nach einem der vorhergehenden Ansprüche, wobei die Sperrelemente (2a, 2b; 3a, 3b) an einer Vorschubhülse (2) vorgesehen sind, welche relativ zu der Zahnstange (1) bewegte werden kann und/oder an einem Führungsstück (3) vorgesehen sind, wobei die Zahnstange (1) relativ zu dem Führungsstück (3) bewegt werden kann.

12. Dosiervorrichtung nach einem der vorhergehenden Ansprüche, wobei die Sperrglieder der Zahnstange (1) über die gesamte Länge der Zahnstange konstant beabstandet entlang der Längsachse der Zahnstange (1) ausgebildet sind.

13. Dosiervorrichtung nach einem der vorhergehenden Ansprüche, wobei die Sperrglieder der Zahnstange (1) in einem ersten Abschnitt entlang der Längsachse der Zahnstange (1) jeweils einen ersten konstanten Abstand voneinander aufweisen und in einem benachbarten zweiten Abschnitt entlang der Längsachse der Zahnstange (1) jeweils einen zweiten konstanten Abstand voneinander aufweisen, wobei der erste Abstand der Sperrglieder in dem ersten Abschnitt und der zweite Abstand der Sperrglieder in dem zweiten Abschnitt unterschiedlich sind.

14. Verfahren zur Vorbereitung der dosierten Verabreichung eines injizierbaren Produktes aus einer Injektionsvorrichtung, wobei eine erste Aufziehbewegung der Injektionsvorrichtung durch die Bewegung eines Einstellmittels oder Knopfes entgegen der Ausschüttrichtung des injizierbaren Produktes durchgeführt wird, wobei durch die Bewegung des Einstellmittels oder Knopfes eine Vorschubhülse (2) entgegen der Ausschüttrichtung des injizierbaren Produktes relativ zu einer Zahnstange (1) bewegt wird, wobei die Zahnstange (1), auf Grund des Anliegens einer Anschlagsflanke mindestens eines Sperrelementes (3a, 3b) eines Führungsstücks (3) an einem Gegenanschlag eines Sperrglieds der Zahnstange (1), in Ruhe bleibt, wobei die Anschlagsflanke mindestens eines Sperrelements (3a, 3b) des Führungsstücks (3) nicht an einem Gegenanschlag eines Sperrglieds der Zahnstange (1) anliegt, wobei nach Beendigung der Aufziehbewegung eine Anschlagsflanke mindestens eines Sperrelements (2a, 2b) der Vorschubhülse (2) so an einem Gegenanschlag eines Sperrglieds der Zahnstange (1) anliegt, dass durch die Bewegung des Einstellelementes oder Knopfes in Ausschüttrichtung des injizierbaren Produktes die Vorschubhülse (2) die Zahnstange (1) in Ausschüttrichtung relativ zu dem Führungsstück (3) bewegt, wobei eine Anschlagsflanke mindestens eines Sperrelements (2a, 2b) der Vorschubhülse (2) nicht an einem Gegenanschlag eines Sperrglieds der Zahnstange (1) anliegt, wobei eine erste Dosis durch die Verschiebung der Zahnstange (1) abgegeben wird und nach der Relativbewegung zwischen zahnstange (1) und Führungsstück (3) eine Anschlagsflanke mindestens eines Sperrelements (3a, 3b) des Führungsstücks (3) an einem Gegenanschlag eines Sperrglieds der Zahnstange (1) anliegt und eine Anschlagsflanke mindestens eines Sperrelements (3a, 3b) des Führungsstücks (3) nicht an einem Gegenanschlag eines Sperrglieds der Zahnstang (1) anliegt.

15. Verfahren zur Vorbereitung der dosierten Verabreichung eines injizierbaren Produktes aus einer Injektionsvorrichtung nach dem vorhergehenden Anspruch, wobei in der Ausgangsposition jedes n-te Sperrelement (2a, 2b) der Vorschubhülse (2) mit seiner Anschlagsflanke an einem Gegenanschlag eines Sperrglieds der Zahnstange (1) anliegt, wobei die weiteren Sperrelemente (2a, 2b) der Vorschubhülse (2) mit ihrer Anschlagsflanke nicht an einem Gegenanschlag eines Sperrglieds der Zahnstange (1) anliegen, und nach dem Aufziehen der Injektionsvorrichtung entgegen der Ausschüttrichtung des injizierbaren Produkts jedes (n+1)-te Sperrelement (2a, 2b) der Vorschubhülse (2) mit seiner Anschlagsflanke an einem Gegenanschlag eines Sperrglieds der Zahnstange (1) anliegt, wobei die weiteren Sperrelemente (2a, 2b) der Vorschubhülse (2) mit ihrer Anschlagsflanke nicht an einem Gegenanschlag eines Sperrglieds der Zahnstange (1) anliegen.

16. Verfahren zur Vorbereitung der dosierten Verabreichung eines injizierbaren Produktes aus einer Injektionsvorrichtung nach einem der beiden vorhergehenden Ansprüche, wobei nach einer Aufziehbewegung der Injektionsvorrichtung jedes n-te Sperrelement (3a, 3b) des Führungsstücks (3) mit seiner Anschlagsflanke an einem Gegenanschlag eines Sperrglieds der Zahnstange (1) anliegt, wobei die weiteren Sperrelemente (3a, 3b) des Führungsstücks (3) mit ihrer Anschlagsflanke nicht an den Gegenanschlag eines Sperrglieds der Zahnstange (1) anliegen, die Zahnstange (1) während des Ausschüttvorganges relativ zu dem Führungsstück (3) bewegt wird und nach dem Ausschüttvorgang jedes (n+1)-te Sperrelement (3a, 3b) des Führungsstücks (3) mit seiner Anschlagsflanke an einem Gegenanschlag eines Sperrglieds der Zahnstange (1) anliegt, wobei die weiteren Sperrelemente (3a, 3b) des Führungsstücks (3) mit ihrer Anschlagsflanke nicht an dem Gegenanschlag eines Sperrglieds der Zahnstange (1) anliegen.

17. Verfahren zur Vorbereitung der dosierten Verabreichung eines injizierbaren Produktes aus einer Injektionsvorrichtung nach einem der drei vorhergehenden Ansprüche, wobei nach jeder Aufziehbewegung durch Bewegung des Einstellelementes oder Knopfes in Ausschüttrichtung des injizierbaren Produktes, konstante Abgabemengen des injizierbaren Produkts ausgeschüttet werden.

**Claims**

1. Dosing device for the dosed administration of an injectable product from an injection device with:

   - a toothed rack (1) with a number of locking members, the locking members along the longitudinal axis of the toothed rack (1) having a first separation (A1) from each other; and
   - at least two locking elements (2a, 2b; 3a, 3b), the locking elements (2a, 2b; 3a, 3b) being arranged in the longitudinal axis of the toothed rack (1) at the same position, in a fixed position ratio relative to each other and in the circumferential direction of the toothed rack (1) and the locking elements (2a, 2b; 3a, 3b) along the longitudinal axis of the toothed rack (1) having a second separation (A2) from each other;

   **characterised in that** the first separation (A1) between the locking members of the toothed rack (1) is not the same as the second separation (A2) between the locking elements (2a, 2b; 3a, 3b).

2. Dosing device according to the preceding claim, in which the first separation (A1) between the locking members of the toothed rack (1) is less than the second separation (A2) between the locking elements (2a, 2b; 3a, 3b).

3. Dosing device according to claim 1, in which the first separation (A1) between the locking members of the toothed rack (1) is larger than the second separation (A2) between the locking elements (2a, 2b; 3a, 3b).

4. Dosing device according to one of the preceding claims, in which the locking elements (2a, 2b; 3a, 3b) in the longitudinal axis of the toothed rack (1) are arranged at a constant separation from each other.

5. Dosing device according to one of the preceding claims in which two, three, four, five, six, seven, eight or more locking elements (2a, 2b; 3a, 3b) are provided.

6. Dosing device according to one of the preceding claims, in which the locking elements (2a, 2b; 3a, 3b) in the longitudinal axis of the toothed rack (1) each have a constant separation from each other, in the longitudinal axis of the toothed rack (1) the stop of every second, third, fourth, fifth or sixth locking element (2a, 2b; 3a, 3b) abuts on a counter-stop of a locking member, and the other locking elements (2a, 2b; 3a, 3b) with their stops do not abut on a counter-stop of a locking member of the toothed rack (1).

7. Dosing device according to one of the preceding claims, in which every $n^{th}$ locking element (2a, 2b; 3a, 3b) with its stop abuts on a counter-stop of a locking member of the toothed rack (1), the other locking elements (2a, 2b; 3a, 3b) with their stops do not abut on a counter-stop of a locking member of the toothed rack (1), the first separation (A1) between the locking members of the toothed rack (1) is less than the second separation (A2) between the locking elements (2a, 2b; 3a, 3b) and the respective separation (A2) between the locking elements (2a, 2b; 3a, 3b) can be represented as a function of the first separation (A1) between the locking members of the toothed rack (1) as:

$$A2 = \frac{n \cdot k + 1}{n} A1$$

   where k and n can be selected from the range of natural numbers.

8. Dosing device according to one of the preceding claims, in which every $n^{th}$ locking element (2a, 2b; 3a, 3b) with its stop abuts on a counter-stop of a locking member of the toothed rack (1), the other locking elements (2a, 2b; 3a, 3b) with their stop do not abut on a counter-stop of a locking member of the toothed rack (1), the first separation (A1) between the locking members of the toothed rack (1) is greater than the second separation (A2) between the locking elements (2a, 2b; 3a, 3b) and the respective separation (A2) between the locking elements (2a, 2b; 3a, 3b) can be represented as a function of the first separation (A1) between the locking members of the toothed rack (1) as:

$$A2 = \frac{n \cdot k - 1}{n} A1$$

   where k and n can be selected from the range of natural numbers.

9. Dosing device according to one of the preceding claims, in which the locking elements (2a, 2b; 3a, 3b) are in the form of inelastic saw-tooth shaped cams with a vertical stop and a slanted slide flank.

10. Dosing device according to one of the preceding claims, in which the locking elements (2a, 2b; 3a, 3b) can be moved radially relative to the toothed rack (1).

11. Dosing device according to one of the preceding claims, in which the locking elements (2a, 2b; 3a, 3b) are provided on a sliding sleeve (2), which can be moved relative to the toothed rack (1) and/or are provided on a guide piece (3), the toothed rack (1) being moveable relative to the guide piece (3).

12. Dosing device according to one of the preceding claims, in which the locking members of the toothed rack (1) are formed over the entire length of the toothed rack at a constant separation along the longitudinal axis of the toothed rack (1).

13. Dosing device according to one of the preceding claims, in which the locking members of the toothed rack (1) in a first section along the longitudinal axis of the toothed rack (1) each have a first constant separation from each other and in an adjacent second section along the longitudinal axis of the toothed rack (1) each have a second constant separation from each other, where the first separation of the locking members in the first section and the second separation of the locking members in the second section are different.

14. Method of preparation of the dosed administration of an injectable product from an injection device, in which a first upward-pulling movement of the injection device is effected by the movement of an adjustment means or button, counter to the discharge direction of the injectable product, while by moving the adjustment means or button, a sliding sleeve (2) is moved counter to the direction of discharge of the injectable product relative to a toothed rack (1), in which the toothed rack (1), due to the abutment of a stop of at least one locking element (3 a, 3b) of a guide piece (3) on a counter-stop of a locking member of the toothed rack (1), remains at rest, while the stop of at least one locking element (3a, 3b) of the guide piece (3) does not abut on a counter-stop of a locking member of the toothed rack (1), while after the completion of the upward-pulling movement a stop of at least one locking element (2a, 2b) of the sliding sleeve (2) abuts on a counter-stop of a locking member of the toothed rack (1) such that by moving the adjustment element or button in the direction of discharge of the injectable product the sliding sleeve (2) moves the toothed rack (1) in the direction of discharge relative to the guide piece (3), while a stop of at least one locking element (2a, 2b) of the sliding sleeve (2) does not abut on a counter-stop of a locking member of the toothed rack (1), while a first dose is administered by displacement of the toothed rack (1) and following the relative displacement between toothed rack (1) and guide piece (3) a stop of at least one locking element (3a, 3b) of the guide piece (3) abuts on a counter-stop of a locking member of the toothed rack (1) and a stop of at least one locking element (3a, 3b) of the guide piece (3) does not abut on a counter-stop of a locking member of the toothed rack (1).

15. Method of preparation of the dosed administration of an injectable product from an injection device according to the preceding claim, in which in the initial position every $n^{th}$ locking element (2a, 2b) of the sliding sleeve (2) with its stop abuts on a counter-stop of a locking member of the toothed rack (1), while the other locking elements (2a, 2b) of the sliding sleeve (2) with their stops do not abut on a counter-stop of a locking member of the toothed rack (1), and after drawing up the injection device counter to the direction of discharge of the injectable product every $(n+1)^{th}$ locking element (2a, 2b) of the sliding sleeve (2) with its stop abuts on a counter-stop of a locking member of the toothed rack (1), while the other locking elements (2a, 2b) of the sliding sleeve (2) with their stop do not abut on a counter-stop of a locking member of the toothed rack (1).

16. Method of preparation of the dosed administration of an injectable product from an injection device according to one of the two preceding claims, in which following an upwards pulling movement of the injection device every $n^{th}$ locking element (3a, 3b) of the guide piece (3) with its stop abuts on a counter-stop of a locking member of the toothed rack (1), while the other locking elements (3a, 3b) of the guide piece (3) with their stops do not abut on the counter-stop of a locking member of the toothed rack (1), the toothed rack (1) is moved relative to the guide piece (3) during the discharge process and following the discharge process every $(n+1)^{th}$ locking element (3a, 3b) of the guide piece (3) with its stop abuts on a counter-stop of a locking member of the toothed rack (1), while the other locking elements (3a, 3b) of the guide piece (3) with their stops do not abut on the counter-stop of a locking member of the toothed rack (1).

17. Method of preparation of the dosed administration of an injectable product from an injection device according to one of the three preceding claims, in which following each upward pulling movement, by moving the adjustment element or button in the direction of discharge of the injectable product, constant dispensing amounts of the injectable product are discharged.

**Revendications**

1. Dispositif de dosage pour l'administration dosée d'un produit injectable à partir d'un dispositif d'injection, comprenant :

    - une crémaillère (1) comprenant une pluralité d'organes de blocage, les organes de blocage présentant un premier intervalle (A1) les uns entre les autres selon l'axe longitudinal de la crémaillère (1) ; et

- au moins deux éléments de blocage (2a, 2b ; 3a, 3b), les éléments de blocage (2a, 2b ; 3a, 3b) étant agencés dans une relation fixe de position les uns par rapport aux autres dans la direction longitudinale de la crémaillère (1) et à la même position dans la direction périphérique de la crémaillère (1), et les éléments de blocage (2a, 2b ; 3a, 3b) présentant un second intervalle (A2) les uns entre les autres selon l'axe longitudinal de la crémaillère (1) ;

**caractérisé en ce que** le premier intervalle (A1) entre les organes de blocage de la crémaillère (1) n'est pas égal au second intervalle (A2) entre les éléments de blocage (2a, 2b ; 3a, 3b).

**2.** Dispositif de dosage selon la revendication précédente, dans lequel le premier intervalle (A1) entre les organes de blocage de la crémaillère (1) est inférieur au second intervalle (A2) entre les éléments de blocage (2a, 2b ; 3a, 3b).

**3.** Dispositif de dosage selon la revendication 1, dans lequel le premier intervalle (A1) entre les organes de blocage de la crémaillère (1) est supérieur au second intervalle (A2) entre les éléments de blocage (2a, 2b ; 3a, 3b).

**4.** Dispositif de dosage selon l'une des revendications précédentes, dans lequel les éléments de blocage (2a, 2b ; 3a, 3b) présentent un intervalle constant les uns entre les autres en direction longitudinale de la crémaillère (1).

**5.** Dispositif de dosage selon l'une des revendications précédentes, dans lequel sont prévus deux, trois, quatre, cinq, six, sept, huit éléments de blocage (2a, 2b ; 3a, 3b) ou plus.

**6.** Dispositif de dosage selon l'une des revendications précédentes, dans lequel les éléments de blocage (2a, 2b ; 3a, 3b) présentent les uns entre les autres un intervalle constant selon la direction longitudinale de la crémaillère, dans la direction longitudinale de la crémaillère (1) le flanc de butée de chaque second, troisième, quatrième, cinquième ou sixième élément de blocage (2a, 2b ; 3a, 3b) est appliqué contre une contrebutée d'un organe de blocage, et les autres éléments de blocage (2a, 2b ; 3a, 3b) ne sont pas appliqués avec leur flanc de butée contre une contrebutée d'un organe de blocage de la crémaillère (1).

**7.** Dispositif de dosage selon l'une des revendications précédentes, dans lequel chaque n-ième élément de blocage (2a, 2b ; 3a, 3b) est appliqué par son flanc de butée contre une contrebutée d'un organe de blocage de la crémaillère (1), les autres éléments de blocage (2a, 2b ; 3a, 3b) ne sont pas appliqués par leur flanc de butée contre une contrebutée d'un organe de blocage de la crémaillère (1), le premier intervalle (A1) entre les organes de blocage de la crémaillère (1) est inférieur au second intervalle (A2) entre les éléments de blocage (2a, 2b ; 3a, 3b), et l'intervalle respectif (A2) entre les éléments de blocage (2a, 2b ; 3a, 3b) en fonction du premier intervalle (A1) entre les organes de blocage de la crémaillère (1) peut être représenté comme :

$$A2 = [(n \cdot k + 1)/\, n]\, A1$$

dans laquelle k et n peuvent être choisis dans l'ensemble des nombres entiers naturels.

**8.** Dispositif de dosage selon l'une des revendications précédentes, dans lequel chaque n-ième élément de blocage (2a, 2b ; 3a, 3b) est appliqué par son flanc de butée contre une contrebutée d'un organe de blocage de la crémaillère (1), les autres éléments de blocage (2a, 2b ; 3a, 3b) ne sont pas appliqués par leur flanc de butée contre une contrebutée d'un organe de blocage de la crémaillère (1), le premier intervalle (A1) entre les organes de blocage de la crémaillère (1) est supérieur au second intervalle (A2) entre les éléments de blocage (2a, 2b ; 3a, 3b), et l'intervalle respectif (A2) entre les éléments de blocage (2a, 2b ; 3a, 3b) en fonction du premierintervalle (A1) entre les organes de blocage de la crémaillère (1) peut être représenté comme :

$$A2 = [(n \cdot k - 1)/\, n]\, A1$$

dans laquelle k et n peuvent être choisis dans l'ensemble des nombres entiers naturels.

**9.** Dispositif de dosage selon l'une des revendications précédentes, dans lequel les éléments de blocage (2a, 2b ; 3a, 3b) sont réalisés sous forme de cames inélastiques en forme de dents de scie avec un flanc de butée vertical et un flanc de coulissement oblique.

**10.** Dispositif de dosage selon l'une des revendications précédentes, dans lequel les éléments de blocage (2a, 2b ; 3a, 3b) peuvent être déplacés en direction radiale par rapport à la crémaillère (1).

**11.** Dispositif de dosage selon l'une des revendications précédentes, dans lequel les éléments de blocage (2a, 2b ; 3a, 3b) sont prévus sur une douille d'avance (2), laquelle peut être déplacée par rapport à la crémaillère (1) et/ou est prévue sur une pièce de guidage (3), la crémaillère (1) pouvant être déplacée par rapport à la pièce de guidage (3).

**12.** Dispositif de dosage selon l'une des revendications précédentes, dans lequel les organes de blocage de la crémaillère (1) sont réalisés sur la totalité de la longueur de la crémaillère à distance constante le long de l'axe longitudinal de la crémaillère (1).

**13.** Dispositif de dosage selon l'une des revendications précédentes, dans lequel les organes de blocage de la crémaillère (1) présentent, dans un premier tronçon selon l'axe longitudinal de la crémaillère (1), un premier intervalle constant les uns entre les autres, et dans un second tronçon voisin selon l'axe longitudinal de la crémaillère (1) ils présentent un second intervalle constant les uns entre les autres, le premier intervalle des organes de blocage dans le premier tronçon et le second intervalle des organes de blocage dans le second tronçon étant différents.

**14.** Procédé pour préparer l'administration dosée d'un produit injectable à partir un dispositif d'injection, dans lequel on exécute un premier mouvement de traction du dispositif d'injection par déplacement d'un moyen de réglage ou d'un bouton à l'encontre de la direction d'évacuation du produit injectable, de sorte que par le déplacement du moyen de réglage ou du bouton une douille d'avance (2) est déplacée à l'encontre de la direction d'évacuation du produit injectable par rapport à une crémaillère (1), ladite crémaillère (1) restant au repos en raison de l'application d'un flanc de butée d'au moins un élément de blocage (3a, 3b) d'une pièce de guidage (3) contre une contrebutée d'un organe de blocage de la crémaillère (1), le flanc de butée d'au moins un élément de blocage (3a, 3b) de la pièce de guidage (3) n'étant pas appliqué contre une contrebutée d'un organe de blocage de la crémaillère (1), de sorte qu'après achèvement du mouvement de traction un flanc de butée d'au moins un élément de blocage (2a, 2b) de la douille d'avance (2) est appliqué contre une contrebutée d'un organe de blocage de la crémaillère (1) de telle façon qu'en raison du mouvement de l'élément de réglage ou du bouton dans la direction d'évacuation du produit injectable, la douille d'avance (2) déplace la crémaillère (1) par rapport à la pièce de guidage (3) dans la direction d'évacuation, un flanc de butée d'au moins un élément de blocage (2a, 2b) de la douille d'avance (2) n'étant pas appliqué contre une contrebutée d'un organe de blocage de la crémaillère (1), de sorte qu'une première dose est fournie par déplacement de la crémaillère (1) et, après le mouvement relatif entre la crémaillère (1) et la pièce de guidage (3), un flanc de butée d'au moins un élément de blocage (3a, 3b) de la pièce de guidage (3) est appliqué contre une contrebutée d'un organe de blocage de la crémaillère (1) et un flanc de butée d'au moins un élément de blocage (3a, 3b) de la pièce de guidage (3) n'est pas appliqué contre une contrebutée d'un organe de blocage de la crémaillère (1).

**15.** Procédé pour préparer l'administration dosée d'un produit injectable à partir d'un dispositif d'injection selon la revendication précédente, dans lequel dans la position de départ chaque n-ième élément de blocage (2a, 2b) de la douille d'avance (2) est appliqué avec son flanc de butée contre une contrebutée d'un organe de blocage de la crémaillère (1), et les autres éléments de blocage (2a, 2b) de la douille d'avance (2) ne sont pas appliqués avec leurs flancs de butée contre une contrebutée d'un organe de blocage de la crémaillère (1) et, après traction du dispositif d'injection en sens contraire à la direction d'évacuation du produit injectable, chaque (n+1)-ième élément de blocage (2a, 2b) de la douille d'avance (2) est appliqué par son flanc de butée contre une contrebutée d'un organe de blocage de la crémaillère (1), et les autres éléments de blocage (2a, 2b) de la douille d'avance (2) ne sont pas appliqués par leurs flancs de butée contre une contrebutée d'un organe de blocage de la crémaillère (1).

**16.** Procédé pour préparer l'administration dosée d'un produit injectable à partir d'un dispositif d'injection selon l'une des deux revendications précédentes, dans lequel après un mouvement de traction du dispositif d'injection chaque n-ième élément de blocage (3a, 3b) de la pièce de guidage (3) est appliqué par son flanc de butée contre une contrebutée d'un organe de blocage de la crémaillère (1), les autres éléments de blocage (3a, 3b) de la pièce de guidage (3) n'étant pas appliqués par leurs flancs de butée contre la contrebutée d'un organe de blocage de la crémaillère (1), la crémaillère (1) est déplacée pendant l'opération d'évacuation par rapport à la pièce de guidage (3), et après l'opération d'évacuation, chaque (n+1)-ième élément de blocage (3a, 3b) de la pièce de guidage (3) est appliqué par son flanc de butée contre une contrebutée d'un organe de blocage de la crémaillère (1), et les autres éléments de blocage (3a, 3b) de la pièce de guidage ne sont pas appliqués par leurs flancs de butée contre la contrebutée d'un organe de blocage de la crémaillère (1).

**17.** Procédé pour préparer l'administration dosée d'un produit injectable à partir d'un dispositif d'injection selon l'une des trois revendications précédentes, dans lequel après chaque mouvement de traction, par déplacement de l'élément de réglage ou du bouton dans la direction d'évacuation du produit injectable, des quantités constantes de produit injectable sont évacuées.

Fig. 1

Fig. 2

Fig. 3c

1 Klick

Fig. 3b

Fig. 3a

Fig. 4

Fig. 5a

Fig. 5b

Fig. 5c

EP 1 855 740 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1228777 B1
- US 2221739 A
- US 20010051792 A1
- US 3517668 A
- WO 0023133 A